# EUROPEAN PATENT APPLICATION

(11) **EP 4 643 857 A1**
(43) Date of publication of application: **05.11.2025**
(21) Application number: 24173383.1
(22) Date of filing: 30.04.2024
(51) Int. Cl.: A61K 31/439, A61K 31/445, A61K 9/00, A61P 25/28

(54) **PHARMACEUTICAL COMBINATION OF DONEPEZIL AND CEVIMELINE**

(71) Applicant: Luka Assets Inc., Orlando, FL 32806 (US)
(72) Inventor: Kuca, Kamil, Hradec Kralove (CZ); Valis, Martin, Hradec Kralove (CZ); Hort, Jakub, Praha (CZ)
(74) Representative: Harber IP s.r.o.

(57) **Abstract**

The present invention relates to combination of donepezil or a pharmaceutically acceptable salt thereof and cevimeline or a pharmaceutically acceptable salt thereof for use in the treatment of Alzheimer's disease. Furthermore, a pharmaceutical formulation containing donepezil or a pharmaceutically acceptable salt thereof, cevimeline or a pharmaceutically acceptable salt thereof, and at least one pharmaceutically acceptable excipient is provided.

## Description

### Field of Art

The invention relates to a pharmaceutical combination of donepezil and cevimeline or salts thereof. The pharmaceutical combination is destined for the treatment of Alzheimer disease.

### Background Art

Alzheimer's disease (AD) is a chronic progressive disease of the nervous system caused by degenerative death of neurons with characteristic histopathological changes. Alzheimer's disease is the most common cause (approx. 60%) of middle-aged and elderly dementia.

The incidence of AD increases exponentially with age, which is also its main risk factor. While the incidence of AD between the ages of 65 and 74 is 3%, it is already 19% between the ages of 75 and 84 and even 47% after the age of 85. With the gradual prolongation of human life, AD is becoming a significant medical as well as socio-economic problem.

AD is characterized by a gradual onset of symptoms, including gradual memory loss, cognitive deficits, inability to reason and make judgments, emotional instability, and loss of spatial orientation. Gradually, personality changes, sleep-wake cycle disorders, and psychotic states may appear. Patients are often incontinent. AD has a continuously progressive character and ends fatally. The median duration from onset of symptoms to death is 8.5 years. It is considered the fourth leading cause of death.

In AD, production of acetylcholine (ACh) in the presynaptic region of the neuron is impaired. Acetylcholine binds to the acetylcholine receptor and functions as a neurotransmitter that mediates the transmission of excitement in the central (CNS) and peripheral nervous system. In the CNS, acetylcholine affects the secretion of many other neurotransmitters (glutamate, glycine, dopamine), so it has a relatively exclusive role among neurotransmitters. Cholinergic transmission in the brain generally affects cognitive functions (memory, attention), so its disorder is one of the causes of Alzheimer's disease and the breakdown of personality.

Currently, AD is incurable, and there is no known treatment that would prevent or reverse the progression of the disease. However, based on the understanding of some mechanisms, drug therapy is available that can slow down the course of AD and alleviate the symptoms of the disease.

As mentioned above, AD is associated with a major loss of cholinergic neurons in the basal ganglia (ncl. basalis Meynerti). Under normal conditions, signaling between these neurons is ensured through the neurotransmitter acetylcholine (ACh), which is released extracellularly into the synaptic cleft and, by binding to another nerve cell, ensures the transmission of signals. Under normal conditions, after fulfilling its function on the postsynaptic membrane, ACh is degraded by the action of the enzyme acetylcholinesterase (AChE).

Considering the importance of the ACh signaling pathway in the cognitive dysfunction present in patients with AD, as well as its connection with other neurological or psychiatric conditions, and the fact that the concentration of acetylcholine in the body of patients with AD is significantly lower than in healthy individuals, increasing the level of acetylcholine, especially in the brain, is considered to alleviate the symptoms of AD. It is possible to achieve this by administering a compound that inhibits the enzyme responsible for the breakdown of ACh, i.e. an acetylcholinesterase inhibitor (AChEi). The consequence of AChE inhibition is then an increased concentration of ACh at the synapse.

There is a continuous need for improvements in AD therapy, and it is the aim of this invention to provide such improvement.

### Disclosure of the Invention

The present invention relates to a combination of donepezil or a pharmaceutically acceptable salt thereof and cevimeline or a pharmaceutically acceptable salt thereof for use in the treatment of Alzheimer's disease (AD), wherein donepezil and cevimeline are administered to a patient simultaneously or sequentially so that donepezil and cevimeline are administered within the interval of one hour. Preferably, donepezil and cevimeline are administered simultaneously. Even more preferably, donepezil and cevimeline are administered in a single unit dose formulation containing both APIs.

Donepezil and cevimeline are preferably co-administered in a weight ratio donepezil or a pharmaceutically acceptable salt thereof : cevimeline or a pharmaceutically acceptable salt thereof within the range from 5:1 to 1:1, more preferably from 3:1 to 1:1, even more preferably 2: 1 to 2:0.8.

In a particularly preferred embodiment, donepezil hydrochloride (optionally in the form of monohydrate) and cevimeline hydrochloride (optionally in the form of hemihydrate) are co-administered in a weight ratio donepezil hydrochloride : cevimeline hydrochloride within the range from 5:1 to 1:1, more preferably from 3:1 to 1:1, even more preferably 2:1 to 2:0.8.

Donepezil, with the IUPAC name (RS)-2-[(1-benzyl-4-piperidyl)methyl]-5,6-dimethoxy-2,3-dihydroinden-1-one, administered usually as hydrochloride, is a selective, reversible, non-competitive inhibitor of AChE (the predominant cholinesterase in the brain). By inhibiting AChE, the hydrolysis of acetylcholine is suppressed, thereby increasing its concentration in the synaptic cleft of neurons. Treatment is started with a dose of 5 mg, and after evaluating the effect after one month of treatment, the dose can be increased to 10 mg per day, which, however, should not be exceeded. It is administered orally. Most side effects are mild and transient in nature and most often include diarrhea, nausea, muscle cramps, fatigue, vomiting and insomnia. Donepezil is currently the standard of treatment for AD.

Donepezil may be used as a base or in the form of its salt with a pharmaceutically acceptable acid. This acid includes inorganic acids such as hydrochloric, sulfuric, nitric, phosphoric or hydrobromic acids; and organic acids such as formic, acetic, hydroxyacetic, propionic, oxalic, succinic, malonic, maleic, mandelic, glycolic, phthalic, lactic, ascorbic, fumaric, malic, tartaric, citric, cyclamic, salicylic, aminosalicylic, pamoic, benzoic, benzenesulfonic, toluenesulfonic, naphthalenesulfonic, ethanesulfonic or methane sulfonic. A particularly preferred pharmaceutically acceptable salt is donepezil hydrochloride, optionally donepezil hydrochloride monohydrate.

Cevimeline, with the IUPAC name (2R,2R)-2'-methylspiro[4-azabicyclo[2.2.2]octane-2,5'-[1,3]oxathiolane, administered usually as hydrochloride hemihydrate, is a cholinergic agonist that binds to muscarinic receptors. It therefore acts on cholinergic receptors in the same way as acetylcholine. Cholinergic transmission in the brain generally affects cognitive functions (memory, attention). Based on these findings, it was hypothesized that cevimeline could have an effect in the treatment of AD and was clinically tested in this indication. However, the clinical evaluation was suspended in phase IIb due to the occurrence of severe dose-dependent adverse effects. Adverse effects are based on its cholinergic effect and the most common are nausea, excessive sweating, runny nose, excessive salivation, rash, headache and blurred vision.

However, cevimeline it has found application in the treatment of other diseases; currently indicated in a dose of 30 mg 3 times a day for the treatment of Sjögren's syndrome. It is administered orally.

Cevimeline may be used as a base or in the form of its salt with a pharmaceutically acceptable acid. This acid includes inorganic acids such as hydrochloric, sulfuric, nitric, phosphoric or hydrobromic acids; and organic acids such as formic, acetic, hydroxyacetic, propionic, oxalic, succinic, malonic, maleic, mandelic, glycolic, phthalic, lactic, ascorbic, fumaric, malic, tartaric, citric, cyclamic, salicylic, aminosalicylic, pamoic, benzoic, benzenesulfonic, toluene sulfonic, naphthalenesulfonic, ethane sulfonic or methanesulfonic. A particularly preferred pharmaceutically acceptable salt is cevimeline hydrochloride (which may be in hemihydrate form).

Donepezil and cevimeline can be in crystalline or amorphous form. The terms "donepezil", "cevimeline" as used herein include amorphs, any polymorphs, hydrates, solvates, and salts, unless the context indicates otherwise. If the compounds can exist as isomers, all isomers such as geometrical isomers, enantiomers, tautomers, etc. are included.

Despite the fact that combinations of various medications were proposed for the treatment of AD, the specific combination of donepezil and cevimeline has never been suggested. Some sources indicate that combination of cevimeline and donepezil would lead to decrease of cognitive and neurological functions. E.g., abstract from Alzheimer's Association International Conference, publikované v Alzheimer's & Dementia®, The Journal of the Alzheimer's association, Volume 14, Issue 7S_Part_26, p. P1373 (available at: https://alz-journals.onlinelibrary.wiley.com/doi/epdf/10.1016/j.jalz.2018.06. 1995), discloses that the application of cevimeline in the treatment of glaucoma competes with the administration of donepezil in AD and is associated with a decrease in cognitive and neurological functions. The combination is thus expected to lead to a completely opposite effect to that which would be needed in the treatment of AD.

Within the framework of the present invention, it was found that co-administration of cevimeline or a pharmaceutically acceptable salt thereof and donepezil or a pharmaceutically acceptable salt thereof, preferably in a ratio disclosed herein, not only has a synergistic effect, but also enables the use of cevimeline in a much smaller amount, thus mitigating the risk of adverse effects. Co-administration of cevimeline with donepezil thus allows to use cevimeline in the treatment of AD and thus increase the options available for the patients suffering from this type of dementia.

The co-administered amount of donepezil or a pharmaceutically acceptable salt thereof can be up to 25 mg per day for an adult. The co-administered amount of cevimeline or a pharmaceutically acceptable salt thereof is calculated so that it corresponds to the weight ratio within the range disclosed herein above.

Preferably, the co-administered amount of donepezil hydrochloride is 3 mg to 10 mg, or 5 mg to 10 mg. Donepezil hydrochloride may be in the form of a monohydrate. The co-administered amount of cevimeline hydrochloride (which may be in hemihydrate form) is calculated so that it corresponds to the weight ratio within the range disclosed herein above.

Cevimeline or a pharmaceutically acceptable salt thereof and donepezil or a pharmaceutically acceptable salt thereof can be administered together or separately, at the same time or at different times (within the interval of one hour). Their simultaneous administration in one conventional pharmaceutical formulation is particularly preferred. Thus, these active ingredients can be prepared in the form of a single dosage form comprising both active ingredients or in the form of separate dosage forms.

The pharmaceutical formulations according to the present invention include forms suitable for oral administration (e.g. powders, granules or mini-tablets, filled into capsules, coated or uncoated tablets, effervescent tablets, immediate-release tablets, orodispersible tablets, tablets with gradual (controlled) release, pills, pellets, and capsules; oral liquid dosages such as syrups and elixirs, suspensions, or emulsions), parenteral (e.g., injectable solutions for intravenous, intramuscular, subcutaneous, or intraperitoneal use), rectal (e.g., suppositories), transdermal (e.g., patches) ) or local (e.g. patches, ointments or gels) administration or for application in the form of implants. The solid, soft or liquid pharmaceutical formulations of the present invention can be produced by methods that are commonly used in the pharmaceutical industry.

Tablets can be prepared by mixing the active ingredients with conventional pharmaceutical carriers such as gelatin, starches, lactose, mannitol, magnesium stearate, cellulose derivatives, and pressing. Capsules can be prepared by mixing active ingredients with inert pharmaceutical fillers or bulking agents and filling hard or soft gelatin capsules with this mixture.

Fillers (diluents) or bulking agents can be microcrystalline cellulose, lactose (anhydrous or in monohydrate form), fructose, glucans, mannitol, sorbitol, lactitol, sucrose or mixtures of the mentioned sugars, powdered cellulose, siliconized microcrystalline cellulose, calcium hydrogen phosphate, calcium carbonate, calcium lactate, or a mixture of the mentioned fillers can be used.

The pharmaceutical formulation may further contain binders such as polyvinylpyrrolidone, microcrystalline cellulose, hydroxyethylcellulose, hydroxypropylcellulose, low substituted hydroxypropylcellulose, hydroxypropylmethylcellulose or other cellulose ethers, tragacanth, guarana gum, starches, pregelatinized starch, polymethacrylate, or a mixture of said binders. According to the present invention, low-substituted hydroxypropyl cellulose means hydroxypropyl cellulose with a content of 5 to 16% by weight of hydroxypropoxy group.

The pharmaceutical formulation may further contain disintegrants such as starches, e.g. pregelatinized starch or corn starch, sodium starch glycolate, cross-linked polyvinylpyrrolidone, microcrystalline cellulose, sodium carboxymethylcellulose, potassium salt, polacrilin, hydroxypropyl cellulose with a low degree of substitution, or a mixture of the aforementioned substances. The disintegration agent can be added to other excipients according to the used method of production of the agent, either during the granulation process and/or during the preparation of the mixture intended for pressing.

The pharmaceutical formulation may further contain glidants/lubricants such as stearic acid, magnesium stearate, calcium stearate, sodium lauryl sulfate, hydrogenated vegetable oil, silicic acid, silicon dioxide, sodium stearyl fumarate, talc or macrogols, or mixtures of the aforementioned substances.

The pharmaceutical formulation may further contain crystallization inhibitors selected from the group of cellulose derivatives, polyvinylpyrrolidone and its derivatives, xanthan gums, pectins, alginates, tragacanth, gum arabic, carrageenans, agar, arabinogalactans, galactomamanns, glucans, etc.

The pharmaceutical formulation may further contain sweeteners, which may be saccharin and its salts, aspartame, cyclamate and its salts, or sugars such as sucrose, lactose and glucose.

The pharmaceutical formulation may further contain dyes, wetting agents and waxes, as well as flavoring agents, which may be natural or synthetic essences.

If necessary, the tablets can also be provided with a coating made of common materials used for the production of coatings.

Liquid compositions suitable for oral administration, such as syrups, elixirs, suspensions or emulsions, are prepared by mixing the active ingredients with solvents (e.g. water, oils, glycerol, propylene glycol, ethanol), suspending agents (e.g. gelatin, carboxymethyl cellulose), emulsifiers ( e.g. sorbitan monooleate), preservatives (e.g. methylparaben or propylparaben), buffering agents (e.g. acetate, phosphate or citrate buffer) dyes, sweeteners and flavourings.

The pharmaceutical formulation for parenteral administration can be prepared in a conventional manner, for example by dissolving the active ingredients in a sterilized aqueous carrier, preferably in water or saline. Preferably, said liquid compositions can further contain wetting agents such as sodium carboxymethylcellulose, methylcellulose, polyvinylpyrrolidone and polyvinyl alcohol, they can also contain a disinfectant (e.g. benzyl alcohol, phenol and thimerosal), a fungicide and optionally an isotonic agent (e.g. sucrose and sodium chloride), local anesthetic, stabilizer and buffer. In order to maintain stability, the drug for parenteral administration may be encapsulated, followed by removal of the liquid medium by conventional lyophilization techniques. The composition can be converted to a liquid composition by dissolving in an aqueous medium.

Soft pharmaceutical formulations, e.g. suppositories, contain active substances uniformly dispersed in a suppository base material, e.g. polyethylene glycol or cocoa butter.

Transdermal pharmaceutical formulations can be single-layered or multi-layered. Multilayer transdermal compositions are formed, for example, by an adhesive matrix layer containing a pharmaceutical formulation, where a backing layer is placed on one side (surface) of the adhesive matrix layer, and a release layer is placed on the other side (surface).

A plastic film such as polyester, polyurethane, polyethylene, polypropylene, nylon, vinyl acetate, polyacrylonitrile or polyethylene terephthalate can be used as the backing layer, or a fabric such as silk, cotton, acrylic, polyester can be used. The matrix layer containing the pharmaceutical formulation contains, in addition to donepezil or a pharmaceutically acceptable salt thereof and cevimeline or a pharmaceutically acceptable salt thereof, an adhesive, such as acrylate or vinyl acetate, and optionally excipients, such as one or more transdermal penetration accelerators. As a release layer, e.g. foil of polystyrene, polyethylene, PVC, polyvinylidene chloride, paper, etc. can be used.

The final dosage forms of the pharmaceutical formulation can be formulated using known methods used in the pharmaceutical industry. The active substance(s) are mixed with pharmaceutically acceptable solid or liquid carriers and/or excipients and the mixture is put into galenic form. Said carriers and excipients, together with methods that are used in the pharmaceutical industry are described in the literature (e.g. Remington's Pharmaceutical Sciences, Edition 18., Mack Publishing Co., Easton, USA, 1990).

The pharmaceutical combination of cevimeline or a pharmaceutically acceptable salt thereof and donepezil or a pharmaceutically acceptable salt thereof according to the present invention shows an excellent therapeutic and prophylactic effect in Alzheimer's disease. This effect is very rapid and the composition has low toxicity, thus it can be administered for a long time.

### Brief Description of Drawings

Figure 1 shows the escape time on individual days of the experiment for the tested groups of laboratory rats.
Figure 2 shows the length of the escape path on the individual days of the experiment in the tested groups of laboratory rats.
Figure 3 shows the escape time on the 4th day of the experiment for individual tested groups of laboratory rats.
Figure 4 shows the escape path on the 4th day of the experiment for individual tested groups of laboratory rats.
Figure 5 shows the escape time on the 5th day of the experiment for individual tested groups of laboratory rats.
Figure 6 shows the escape path on the 5th day of the experiment for individual tested groups of laboratory rats.

### Examples

### Example 1: Pharmaceutical formulation

The following compositions of pharmaceutical formulation can serve as examples of the present invention:

| | mg/tablet | mg/tablet | mg/tablet | mg/tablet |
|---|---|---|---|---|
| Donepezil HCl | 5 | 10 | 15 | 15 |
| Cevimeline HCl | 2.5 | 5 | 6 | 10 |
| Mannitol (diluent) | - | - | 190 | 190 |
| Lactose monohydrate (diluent) | 92,5 | 185 | - | - |
| Microcrystalline cellulose (diluent) | 17,5 | 35 | 38 | 38 |
| Hydroxypropyl cellulose (binder) | 3,7 | 7,4 | 8 | 8 |
| Magnesium stearate (lubricant) | 3,7 | 7,4 | 8 | 8 |
| Corn starch(desintegrant) | 19 | 38 | 41 | 41 |

Donepezil HCl may be used in monohydrate form, cevimeline HCl may be used in hemihydrate form. The tablets are prepared by mixing and wet granulation of components without the lubricant, wherein the lubricant is mixed with the resulting granulate and the mixture is pressed into tablets. The tablets may optionally be coated, e.g. by macrogol-based coating (macrogol, talc, optionally iron oxide pigment).

### Example 2

The effect of the pharmaceutical combination of donepezil and cevimeline was tested in laboratory rats. The Morris water maze (MWM) test was used.

The Morris water maze is a widely used testing method in behavioral neuroscience. It enables the study of spatial learning and memory in laboratory rats with great precision and can also be used to evaluate damage to specific brain regions. The Morris Water Maze is a circular pool in which animals learn to find a hidden island just below the surface that will allow them to escape from the pool. Its position is fixed. To search for an island, laboratory rats use various landmarks in the surrounding space. The time within which the animal is able to find this island and the distance that the given laboratory rat swims to the island are measured.

Testing in the Morris Water Maze took place over five consecutive days. The tested compounds and scopolamine were administered daily according to the dosage schedule shown below. Scopolamine is a parasympatholytic that blocks muscarinic receptors for acetylcholine. Its administration causes memory disorders and impaired judgment. In this experiment, its administration causes a cognitive deficit.

Administered compounds:
- donepezil hydrochloride (in monohydrate form) (done) in a dose of 2 mg/kg donepezil HCl, applied i.p. (intraperitoneally) 40 min before the experiment
- cevimeline hydrochloride (in hemihydrate form) (cevi) in a dose of 0.9 mg/kg cevimeline HCl, applied i.p. 20 min before the experiment.
- scopolamine (scop) in a dose of 2.5 mg/kg, applied i.p. 20 min before the experiment

Each test group contained 6 laboratory rats. The test groups were as follows:
Group 1: physiological solution only (sal) = control group ("healthy" laboratory rats).
Group 2: scopolamine only (scop) = laboratory rats with a cognitive deficit but with no treatment.
Group 3: donepezil-scopolamine (done-scop) = laboratory rats with a cognitive deficit, donepezil only treatment.
Group 4: cevimelin-scopolamine (cevi-scop) = laboratory rats with a cognitive deficit, cevimeline only treatment.
Group 5: donepezil-cevimelin-scopolamine (done-cevi-scop) = laboratory rats with a cognitive deficit, donepezil+cevimeline treatment.

Each laboratory rat underwent 8 test swims lasting 60 seconds each day. On the fifth day, a probe trial took place.

### Results:

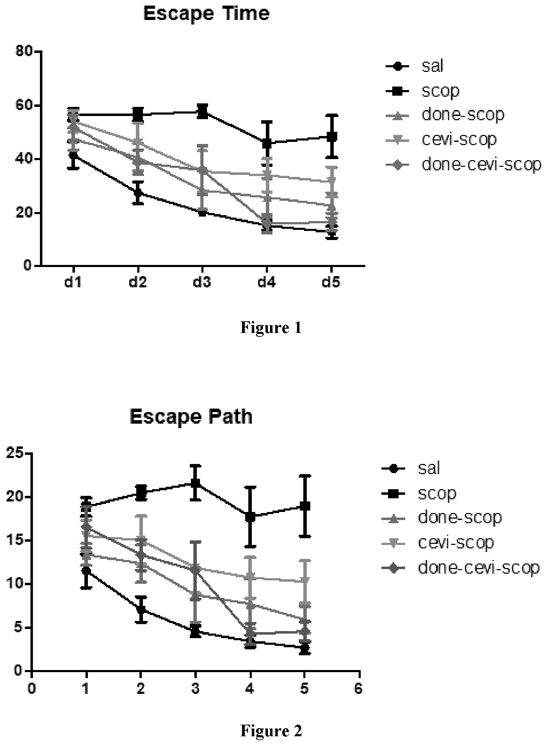

Laboratory rats receiving saline solution (sal, Group 1) represent a control group - i.e. "healthy" individuals. Both the time and the length of the escape path decrease over the course of the five-day experiment, which corresponds to intact learning ability and spatial memory. Lab rats find the island more easily during the experiment. In contrast, laboratory rats administered scopolamine (scop, Group 2) alone are cognitively deficient animals. They are not able to remember the location of the island, so the time and escape path at the end of the experiment did not differ from the initial time and escape path. Other groups were administered scopolamine and the tested substances either in monotherapy or in combined treatment. The combination of donepezil + cevimeline showed a beneficial effect on both monitored parameters - escape path and escape time - on the fourth and fifth day of the experiment. Donepezil alone showed a beneficial effect on the fifth day (Fig. 1 and 2).

Fig. 1 shows the escape time on individual days of the experiment for the test groups. No progress was observed in cognitively deficient laboratory rats (scop). Significant reduction in escape time was observed in "healthy" laboratory rats (sal) and donepezil+cevimeline (done-cevi) administered laboratory rats, a slighter effect was observed with donepezil alone, especially on days 4 and 5 of the experiment.

Fig. 2 shows the length of the escape path on individual days of the experiment for the test groups. No progress was observed in cognitively deficient laboratory rats (scop). Significant shortening of the escape route was observed in "healthy" laboratory rats (sal) and laboratory rats with the applied done-cevi combination, slighter effect with donepezil alone, especially on days 4 and 5 of the experiment.

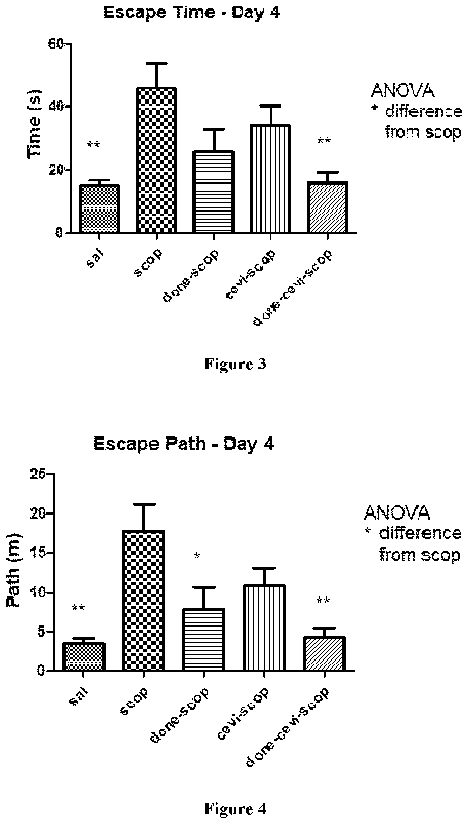

On the fourth day of testing, the escape route and time of laboratory rats that received scopolamine and done-cevi combination were comparable to the results of laboratory rats administered saline (sal). Thus, the laboratory rats "treated" with the done-cevi combination achieved the same results as the "healthy" laboratory rats (the number "*" in the graph indicates the significance of the difference in the result compared to the scopolamine alone - laboratory rats with a cognitive deficit, see Fig. 3 and 4).

Fig. 3 shows the escape time on the 4th day of the experiment for individual test groups. The done-cevi combination in cognitively deficient laboratory rats has shown a beneficial medical effect and the results are comparable to "healthy" laboratory rats (sal).

Fig. 4 shows the escape path on the 4th day of the experiment for individual test groups. The done-cevi combination in cognitively deficient laboratory rats has shown a beneficial medical effect and the results are comparable to "healthy" laboratory rats. A certain effect, however smaller than with the combination treatment, was also observed with donepezil alone.

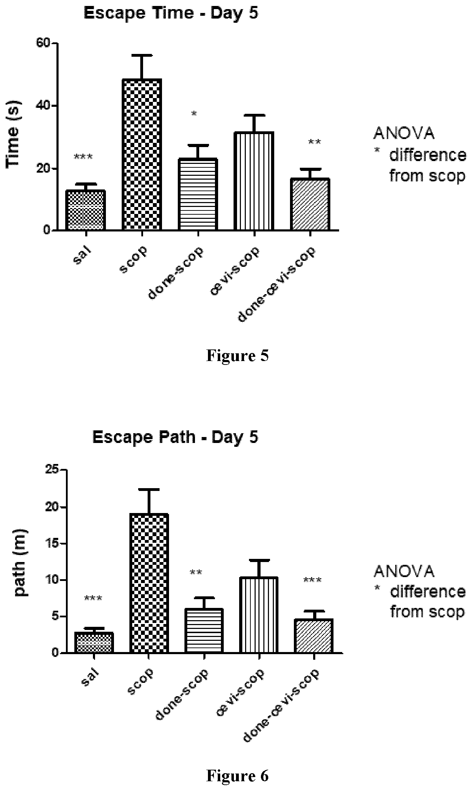

The beneficial effect of the combination of done-cevi administration and donepezil alone was maintained on the fifth day of the experiment (see Figs. 5 and 6), although in comparison with the "healthy" laboratory rats (sal) the effect was lower than on the 4th day.

Fig. 5 shows the escape time on the 5th day of the experiment for individual test groups. The beneficial medical effect of the done-cevi combination continues, however, the result is not as good as in laboratory rats with applied physiological solution. A lower effect was observed with donepezil alone.

Fig 6 shows the escape path on the 5th day of the experiment for individual test groups. The results of the done-cevi combination are comparable to the results in laboratory rats with applied physiological solution (sal). Beneficial effect of donepezil alone was also recorded, but not as significant as with the combination treatment done-cevi.

### Industrial applicability

The invention can be used in the treatment of Alzheimer's disease.

## Claims

1. Combination of donepezil or a pharmaceutically acceptable salt thereof and cevimeline or a pharmaceutically acceptable salt thereof for use in the treatment of Alzheimer's disease.

2. Combination for use according to claim 1, wherein donepezil or a pharmaceutically acceptable salt thereof and cevimeline or a pharmaceutically acceptable salt thereof are administered to a patient simultaneously or sequentially so that donepezil and cevimeline are administered within the interval of one hour.

3. Combination for use according to claim 1 or 2, wherein donepezil and cevimeline are co-administered in a weight ratio donepezil or a pharmaceutically acceptable salt thereof : cevimeline or a pharmaceutically acceptable salt thereof within the range from 5:1 to 1:1, more preferably from 3:1 to 1:1, even more preferably 2: 1 to 2:0.8.

4. Combination for use according to claim 1 or 2, wherein donepezil hydrochloride and cevimeline hydrochloride are co-administered in a weight ratio donepezil hydrochloride : cevimeline hydrochloride within the range from 5:1 to 1:1, more preferably from 3:1 to 1:1, even more preferably 2: 1 to 2:0.8.

5. A pharmaceutical formulation containing donepezil or a pharmaceutically acceptable salt thereof, cevimeline or a pharmaceutically acceptable salt thereof, and at least one pharmaceutically acceptable excipient.

6. The pharmaceutical formulation according to claim 5, containing donepezil and cevimeline in a weight ratio donepezil or a pharmaceutically acceptable salt thereof : cevimeline or a pharmaceutically acceptable salt thereof within the range from 5:1 to 1:1, more preferably from 3:1 to 1:1, even more preferably 2:1 to 2:0.8.

7. The pharmaceutical formulation according to claim 5, containing donepezil hydrochloride and cevimeline hydrochloride in a weight ratio donepezil hydrochloride : cevimeline hydrochloride within the range from 5:1 to 1:1, more preferably from 3:1 to 1:1, even more preferably 2: 1 to 2:0.8.

8. The pharmaceutical formulation according to any one of claims 5 to 7, containing up to 25 mg donepezil or a pharmaceutically acceptable salt thereof in unit dose.

9. The pharmaceutical formulation according to any one of claims 5 to 7, containing 3 mg to 10 mg, preferably 5 mg to 10 mg, of donepezil hydrochloride.

10. The pharmaceutical formulation according to any one of claims 5 to 9 for use in the treatment of Alzheimer's disease.
